# FASCICULE DE BREVET EUROPEEN

(11) **EP 1 954 251 B1**
(45) Date de publication et mention de la délivrance du brevet: **07.03.2012**
(21) Numéro de dépôt: 06831163.8
(22) Date de dépôt: 23.11.2006
(51) Int. Cl.: A61K 9/50, A61K 33/26, A61P 7/06

(54) **COMPOSITION A LIBERATION PROLONGEE DE SEL DE FER EN TANT QU'ACTIF, SON PROCEDE DE PREPARATION ET SON UTILISATION**
ZUSAMMENSETZUNG MIT LANGSAMER FREISETZUNG VON FE SALZEN, VERFAHREN ZU IHRER HERSTELLUNG UND IHRE VERWENDUNG
SLOW-RELEASE COMPOSITION OF FE SALT AS ACTIVE INGREDIENT, METHOD FOR THE PREPARATION THEREOF AND USE OF THE SAME

(30) Priorité: 01.12.2005 FR 0512189
(43) Date de publication de la demande: 13.08.2008
(73) Titulaire: PIERRE FABRE MEDICAMENT, 92100 Boulogne (FR)
(72) Inventeur: BERTHOUMIEU, Didier, F-31290 Villenouvelle (FR); DUPINAY, Pierre, F-31460 Toutens (FR); TRANNOY, Philippe, F-31000 Toulouse (FR)
(74) Mandataire: Rousseau, Pierrick Edouard
(86) Numéro de dépôt international: PCT/FR2006/002577
(87) Numéro de publication internationale: WO 2007/063200

(56) Documents cités:
- WO-A-01/15665
- FR-A- 2 792 527
- US-A1- 2002 034 544
- US-A1- 2003 035 837
- DEGUSSA: "Eudragit"[Online] janvier 2005 (2005-01), XP002399809 Extrait de l'Internet: URL:http://www.rohacell.com/de/pharmapolym ers?content=/de/pharmapolymers/service/lit erature/praktikum> [extrait le 2006-09-20]

## Description

La présente invention concerne une composition pharmaceutique ou nutraceutique pour la libération prolongée de sels de fer, son procédé de préparation et son utilisation en thérapeutique. Les sels de fer sont plus particulièrement du sulfate ferreux.

Le fer est parmi tous les nutriments un de ceux dont la couverture des besoins pose, dans l'espèce humaine, le plus de problème pratique à résoudre. La carence en fer constitue un trouble nutritionnel très répandu dans le monde qui concerne en tout premier lieu les pays en voie de développement mais également, à un degré d'intensité certes moindre, les pays industrialisés. Quelque soit le niveau de développement, certains groupes de populations sont particulièrement exposés au risque de développer une carence en fer : il s'agit principalement des femmes en âges de procréer et notamment les femmes enceintes, les jeunes enfants et les adolescents.

Le fer est présent en très petite quantité dans l'organisme (50mg/kg chez l'homme, 35mg/kg chez la femme). Une partie de ce fer est utilisée chaque jour pour satisfaire les besoins de l'organisme, ceux-ci variant au cours de la vie. Pour maintenir un statut en fer adéquat, il faut compenser les pertes par des apports alimentaires. Sinon, un déséquilibre s'installe avec comme conséquences, en fonction du degré modéré ou du stade avancé de la carence en fer, une perte d'énergie, une baisse des performances physiques et intellectuelles, une sensibilité accrue aux infections, des perturbations au cours de la gestation... Cette balance peut être déséquilibrée dans le sens de la carence en diverses circonstances : insuffisance des apports ou diminution de l'absorption, augmentation des pertes, augmentation des besoins. Une des conséquences cliniques de la carence en fer est l'anémie par carence martiale (ou anémie ferriprive) aux effets délétères bien connus. Il est donc nécessaire d'assurer un apport en fer suffisant et adapté à chaque individu par l'alimentation, mais aussi par des compléments nutritionnels et des médicaments.

Cependant, le fer est peu absorbé par l'organisme (résorption intestinale faible de 10 à 20%). Son profil de libération doit donc être prolongé pour s'affranchir de cette faible résorption.

Les méthodes aujourd'hui utilisées et connues de l'homme du métier sont principalement des méthodes d'encapsulation des sels de fer par un polymère, une gélatine, de l'amidon..., des méthodes de dissolution des sels de fer dans une matrice polymère ou l'association de ces deux méthodes. On rencontre également des formes bioadhésives qui permettent de bloquer la forme sur son site de résorption. L'augmentation de la durée de contact permet d'augmenter les concentrations locales et donc les quantités qui pénètrent. La nature des polymères peut être très variée (dérivés de cellulose, résines méthacryliques...) et dépend du profil de libération recherché. Le brevet US6402997 couvre une méthode de préparation de microcapsules à base de fer soluble par l'utilisation d'un ester d'acide gras (monostéarate polyglycérine), ces microcapsules sont utilisées dans le domaine des compléments alimentaires.

La demande de brevet WO03/055475 concerne une formulation pharmaceutique à libération contrôlée contenant de la venlafaxine : cette formulation se présente sous la forme d'un noyau comprenant la venlafaxine et divers polymères cellulosiques; ledit noyau étant enrobé avec un enrobant polymérique constitué d'au moins deux polymères. Cette composition comprend de nombreux (au moins quatre) et divers polymères, dans des ratios particuliers et différents dans le noyau et l'enrobage ; ce qui rend le procédé de préparation long et fastidieux.

Actuellement, la spécialité TARDYFERON^{Ⓡ} commercialisée par les laboratoires Pierre Fabre Médicament est indiquée pour le traitement des carences en fer. Ce produit à base de sulfate ferreux présente les avantages d'être très bien toléré, stable dans le temps (pas de phénomène d'oxydation du fer ferreux), et libéré de façon prolongée dans l'organisme. Cependant, un des excipients utilisés est la mucoprotéose d'origine animale. Et c'est précisément cette mucoprotéose qui contribue au profil de libération obtenu et à la protection du fer ferreux contre l'oxydation. Mais l'utilisation d'une matière animale engendre des inconvénients incontournables tels que des problèmes de sécurité microbiologique, de sécurité virale, et d'éventuelles difficultés d'approvisionnement.

Par conséquent, il apparaît nécessaire de disposer d'une nouvelle formulation à libération prolongée des sels de fer pour s'affranchir des problèmes liés à l'utilisation d'une matière animale dans une composition pharmaceutique ou nutraceutique.

A cette fin, la présente invention concerne une composition pharmaceutique ou nutraceutique à base de sels de fer associés à une combinaison d'excipients, en remplacement de la mucoprotéose, permettant d'assurer la libération prolongée desdits sel de fer.

La présente invention concerne également son procédé de préparation et son utilisation pour la préparation d'un produit pharmaceutique ou nutraceutique à libération prolongée de sels de fer.

La composition pharmaceutique ou nutraceutique à libération prolongée de sels de fer selon la présente invention comprend au moins un granulé enrobé ; ledit granulé enrobé étant composé d'une particule comprenant lesdits sels de fer enrobée d'au moins deux enveloppes caractérisées en ce qu'elles comprennent une combinaison d'excipients composée :
- d'au moins un copolymère (a) : poly(ethyl acrylate-co-methyl methacrylate-co-trimethylammonioethyl methacrylate chloride) 1:2:0.1 (Eudragit RS30D^{®}) présentant un pourcentage molaire de groupes ammonium quaternaire inférieur ou égal à 8%,
- en association avec un second copolymère (b) : poly(ethyl acrylate-co-methyl methacrylate-co-trimethylammonioethyl methacrylate chloride) 1:2:0.2 (Eudragit RL30D^{®}) présentant un pourcentage molaire de groupes ammonium quaternaire supérieur à 8%,
- dans un ratio massique (a) / (b) compris entre 60/40 et 80/20,
- et avec une quantité de (a) comprise entre 2,5% et 5,0% en poids sec du poids total de la composition.

Les groupements ammonium quaternaire contenus dans les copolymères (a) et (b) sont présents dans des quantités différentes ; ce qui confère aux enveloppes obtenues avec ces polymères des propriétés de perméabilité différentes. Le copolymère (a) seul, forme des enveloppes présentant une faible perméabilité alors que le copolymère (b) seul, forme des enveloppes avec une forte perméabilité.

On a maintenant remarqué de manière intéressante que le choix des excipients et particulièrement des deux polymères (a) et (b) dans un ratio particulier permet d'obtenir une composition présentant un profil de libération prolongée des sels de fer en vue d'une utilisation pour un traitement thérapeutique à visée pharmaceutique ou nutraceutique, et plus particulièrement en vue d'une utilisation pour le traitement et/ou la prévention de carences en sels de fer. En outre, on a constaté de façon inattendue et surprenante que ladite composition présente également une bonne stabilité, et assure une protection des sels de fer par rapport à l'oxydation.

Avantageusement, le ratio massique (a) / (b) des deux polymères selon la présente invention est de l'ordre de 70/30, et encore plus avantageusement de l'ordre de 65/35. De façon avantageuse, la quantité massique du copolymère (a) est comprise entre environ 3,5% et environ 4,0% en poids sec du poids total de la composition, et est préférentiellement égale à environ 3,9% en poids sec du poids total de la composition.

Le pourcentage molaire des groupements ammonium quaternaire dans le copolymère (a) selon la présente invention est préférentiellement compris entre environ 2% et environ 8%, de façon plus préférentielle entre environ 4% et environ 6%, et de façon encore plus préférentielle égal à environ 5%.

Le pourcentage molaire des groupements ammonium quaternaire dans le copolymère (b) selon la présente invention est préférentiellement compris entre environ 8% et environ 12%, de façon plus préférentielle entre environ 9% et environ 11%, et de façon encore plus préférentielle égal à environ 10%.

L'invention sera mieux comprise et les buts, avantages et caractéristiques de celle-ci apparaîtront plus clairement de la description qui suit et qui est faite en référence aux dessins annexés représentant des exemples non limitatifs de réalisation de l'invention sur lesquels :

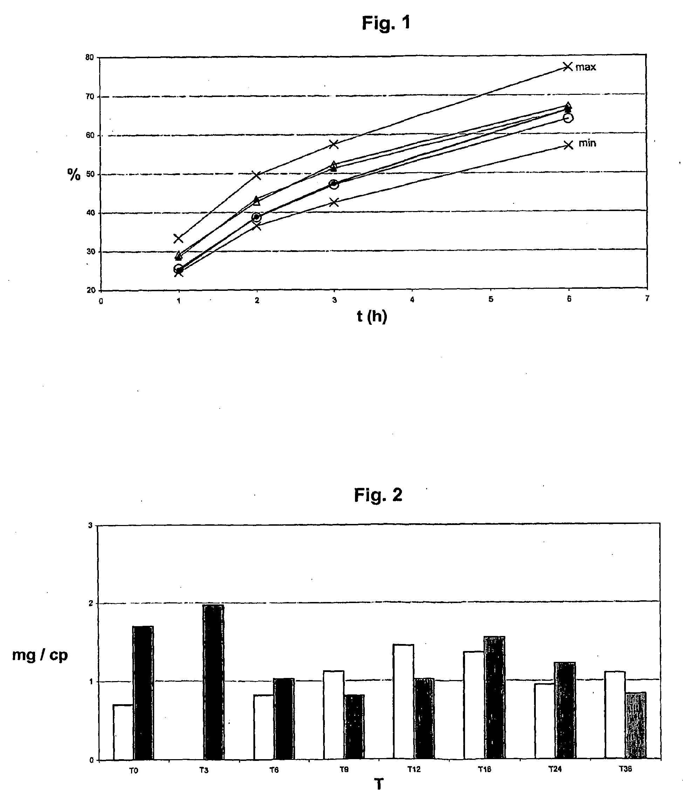

La figure 1 représente le pourcentage de dissolution du sulfate ferreux en fonction du temps (les points de mesure ont été choisis à 1, 2, 3 et 6 heures); pour les deux lots G93 (Δ) et G94 (▲) préparés selon l'exemple 1 de la présente invention et les deux lots G6406 (•) et G6372 (o) de TARDYFERON^{Ⓡ} avec mucoprotéose. Sont également représentées, les spécifications (x) minimales et maximales relatives à la spécialité pharmaceutique de référence TARDYFERON^{®}. On observe que les profils de dissolution du fer ferreux dans des compositions selon la présente invention sont très similaires à ceux obtenus avec deux lots de TARDYFERON^{Ⓡ} . On en conclut que la composition selon la présente invention permet d'obtenir le profil de libération souhaité.

La composition telle que décrite par la présente invention permet également de conserver, et ceci de manière surprenante, les paramètres de protection des sels de fer contre le phénomène d'oxydation. En effet, le point tout à fait remarquable de l'invention est qu'aucun anti-oxydant n'est nécessaire pour assurer la stabilité des sels de fer.

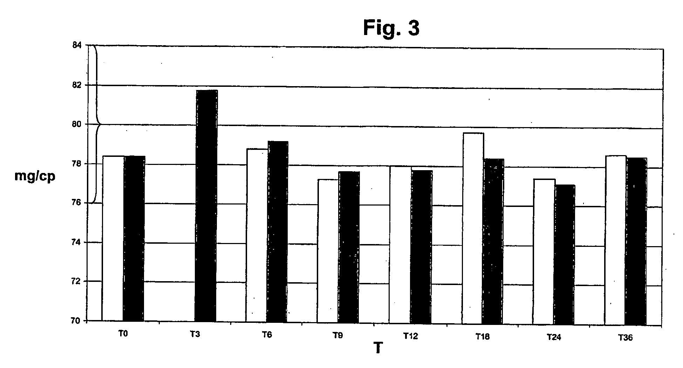

Les figures 2 et 3 schématisent les résultats de stabilité obtenus sur 36 mois pour les deux lots préparés selon l'exemple 1 de la présente invention : les figures 2 et 3 concernent respectivement le dosage du fer ferrique et le dosage du fer total.

La figure 2 représente le dosage du fer ferrique (produit suite à l'oxydation du fer ferreux) dans le temps : les mesures du fer ferrique, en mg par comprimé, ont été effectuées à 0, 3, 6, 9, 12, 18, 24 et 36 mois à partir de comprimés issus des mêmes lots G93 ( 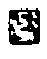 ) et G94 (■) que précédemment. On observe ainsi que les résultats obtenus sont tous inférieurs à la limite maximale de 3,0 mg/comprimé imposée dans la spécification relative à la spécialité pharmaceutique de référence TARDYFERON^{Ⓡ} et qu'il n'y a donc pas de transformation significative de fer ferreux en fer ferrique dans les compositions selon la présente invention. Ce résultat met en évidence l'effet protecteur inattendu de la composition utilisée contre l'oxydation.

C'est enfin la bonne stabilité dans le temps de ces deux mêmes lots G93 (■) et G94 (■) qui a été mise en évidence dans la figure 3. En effet, le fer total, exprimé en mg par comprimé, contenu dans ces comprimés a été dosé à différents temps T (à 0, 3, 6, 9, 12, 18, 24 et 36 mois). Les spécifications relatives à la spécialité pharmaceutique de référence TARDYFERON^{®} indiquent que la quantité de fer doit être comprise entre 76 et 84 mg par comprimé. On observe que l'ensemble des dosages effectués est bien compris dans cette gamme de valeurs. On en conclut qu'il n'y a pas de perte significative en fer total dans les compositions selon la présente invention.

De plus, par rapport à l'utilisation de la mucoprotéose d'origine animale, les avantages d'une formule excipiendaire selon la présente invention, sont notamment :
- l'amélioration de la sécurité du produit, notamment la sécurité virale ;
- l'amélioration de la faisabilité industrielle en rapport avec la disponibilité facilitée des matières premières ;
- un procédé industriel sécurisé puisqu'il ne nécessite plus de travaux d'extraction à partir de matières premières d'origine animale pour l'obtention de la mucoprotéose.

Un autre avantage de la composition selon la présente invention réside dans le fait que ladite composition nécessite qualitativement et quantitativement moins d'excipients que la spécialité TARDYFERON^{Ⓡ}, ce qui se traduit dans ce cas par un coût de revient moindre.

Les figures précédentes illustrent les résultats obtenus à partir d'une composition selon la présente invention à base de sulfate ferreux comme seule source de sels de fer. Cependant, on peut raisonnablement envisager que les bénéfices de la présente invention puissent être étendus à toute composition renfermant des sels de fer pharmaceutiques, nutraceutiques, cosmétique ou vétérinaire auquel on souhaite associer un profil de libération prolongée.

De façon avantageuse, les sels sont sous forme de fer II ou III. De façon plus avantageuse, les sels de fer sont choisis dans le groupe comprenant le sulfate, le fumarate, le gluconate, l'ascorbate, l'oxalate, le succinate, le glycérophosphate et le férédétate.

De façon encore plus avantageuse, les sels de fer sont du sulfate ferreux.

Avantageusement, la composition comprend une ou plusieurs substances pharmaceutiquement ou nutraceutiquement acceptables choisi parmi les diluants, les liants, les lubrifiants, les anti-agglomérants, les plastifiants utilisés seuls ou en mélange.

Le plastifiant est avantageusement dans ce cas choisi dans le groupe formé de l'acétyl tributyl citrate, acétyl triéthyl citrate, glycérides d'acides gras acétylés, huile de ricin, diéthyl sébacate, dibutyl sébacate, glycérol, monostéarate de glycérol, glycéryl triacétate, polyéthylène glycols, copolymères polyoxyéthylène/polyoxypropylène, propylène glycol, tributyl citrate, triéthylcitrate, utilisés seuls ou en mélange et préférentiellement du triéthylcitrate.

De façon avantageuse, le diluant est choisi dans le groupe formé des celluloses et de lactose; préférentiellement une cellulose.

Avantageusement, le liant est choisi parmi les maltodextrines ou les povidones ; préférentiellement une maltodextrine.

La présente invention concerne également l'utilisation de la composition selon la présente invention dans une formulation pharmaceutique ou nutraceutique à libération prolongée des sels de fer. Plus particulièrement, la présente invention concerne l'utilisation d'une telle formulation dans le traitement et/ou la prévention des carences en sels de fer.

Le terme « pharmaceutique » au sens de la présente invention comprend les formulations pour la préparation de médicaments destinés à un traitement préventif ou curatif en médecine humaine et aussi en médecine animale.

Plus particulièrement la composition selon la présente invention est utilisée pour la préparation d'un médicament destiné au traitement et/ou à la prévention des carences en fer avec ou sans anémie.

De façon plus particulière, la carence en fer est une anémie par carence martiale. Et de façon encore plus particulière, l'anémie par carence martiale est rencontrée chez la femme enceinte ; et il y a recours à une telle utilisation lorsqu'un apport alimentaire suffisant en fer ne peut être assuré.

De façon avantageuse, la composition selon la présente invention est caractérisée en ce qu'elle se présente sous forme orale.

La forme orale est préférentiellement choisie parmi des comprimés, des gélules, des capsules, des pastilles à sucer, des poudres pour suspensions buvables et sirops.

La présente invention concerne enfin un procédé de préparation de granulés enrobés pour une formulation pharmaceutique ou nutraceutique selon la présente invention, caractérisé en ce qu'il comprend les étapes suivante :
1) Enrobage des sels de fer avec une dispersion aqueuse des deux copolymères (a) et (b), dont le ratio massique (a) / (b) est compris entre 60/40 et 80/20, et avec une quantité de (a) comprise entre 2,5% et 5,0% en poids sec du poids total de la composition ; dans un mélangeur à pâles,
2) Emottage des granulés obtenu en 1),
3) Séchage des granulés obtenus en 2), jusqu'à l'obtention d'une humidité résiduelle inférieure ou égale à 3,5%,
4) Enrobage en lit d'air fluidisé des granulés séchés obtenus en 3) par pulvérisation d'une dispersion aqueuse des deux copolymères (a) et (b), dont le ratio massique (a) / (b) est compris entre 60/40 et 80/20, et avec une quantité de (a) comprise entre 2,5% et 5,0% en poids sec du poids total de la composition,
5) Séchage des granulés enrobés obtenus en 4), jusqu'à l'obtention d'une humidité résiduelle inférieure ou égale à 3,5%,
6) Calibrage et égalisation des granulés secs.

Une caractéristique fondamentale du procédé de la présente invention réside dans le fait qu'il emploie uniquement de l'eau comme solvant. Ceci facilite la mise en oeuvre dudit procédé et préserve les opérateurs, lors de la fabrication, des dangers potentiels liés à l'utilisation de solvants organiques.

Le procédé de la présente invention implique donc deux étapes principales 1/ et 4/. Ce double enrobage des particules de sels de fer conditionne le profil de libération desdits sels de fer.

Par « enrobage », on entend au sens de la présente invention le dépôt d'une couche de filmogène sur une particule renfermant notamment les sels de fer.

Les étapes 1/ à 3/ permettent d'obtenir un premier granulé caractérisé en ce que l'humidité résiduelle du mélange à la fin de l'étape 3/ est inférieure ou égale à environ 3,5% et préférentiellement comprise entre environ 2,5% et environ 3,5%. Avantageusement, le mélangeur utilisé au cours de l'étape 1/ est un mélangeur de type FIELDER^{Ⓡ} ou équivalent connu de l'homme du métier. Dans un mode particulier de réalisation de l'invention, l'étape 2 d'émottage est réalisée sur un granulateur oscillant muni d'une grille dont l'ouverture de maille est définie en fonction des spécificités voulues. L'étape suivante de séchage peut être réalisée de différentes manières connues de l'homme du métier : plus particulièrement le séchage est effectué en lit d'air fluidisé.

Les étapes 4/ et 5/ permettent d'obtenir un deuxième granulé caractérisé en ce que l'humidité résiduelle du mélange à la fin de l'étape 5/ est inférieure ou égale à environ 3,5% et préférentiellement comprise entre environ 2,5% et environ 3,5%.

De façon avantageuse, le calibrage 6/ est effectué sur un granulateur oscillant muni d'une grille dont l'ouverture de maille est définie en fonction des spécificités voulues.

On peut envisager de conditionner directement les granulés ainsi obtenus à la fin de l'étape 6/ sous forme de poudre pour suspension buvable ou pour sirop par exemple.

Dans un mode particulier de réalisation de l'invention, les granulés obtenus à la fin de l'étape 6/sont lubrifiés. Ainsi suite à la calibration, on introduit dans le granulateur oscillant au moins une substance lubrifiante choisie parmi les lubrifiants couramment mis en oeuvre dans les domaines pharmaceutique et nutraceutique, plus particulièrement, la substance lubrifiante est un mélange de talc et de dibéhénate de glycérol.

On peut alors envisager de conditionner ces granulés ci-dessus sous forme de comprimés, de gélules ou de pastilles à sucer par exemple. Dans ce cas, une étape supplémentaire de compression de ces mêmes granulés est nécessaire pour l'obtention de comprimés ou de pastilles à sucer.

Dans un mode particulier de réalisation de l'invention, le ratio massique (a) / (b) des copolymères (a) et (b) est identique au cours des étapes 1/ et 4/ du procédé selon la présente invention.

Il est également envisageable d'effectuer l'étape 4/du procédé de la présente invention plusieurs fois successivement et obtenir ainsi un système « multicouches », c'est à dire constitué d'un granulé de sels de fer selon les étapes 1/ à 3/ puis une succession de couches selon l'étape 4/. Ce système sera utilisé en fonction du profil de libération des sels de fer recherchés.

Les exemples suivants sont donnés à titre indicatif non limitatif.

### Exemple 1 Composition pharmaceutique selon la présente invention à base de sulfate ferreux.

| **Matières premières** | **Formule unitaire : pour 1 comprimé** |
|---|---|
| Sulfate ferreux | qs pour 80mg de fer élément |
| Maltodextrine | 25,00 mg |
| Cellulose microcristalline | qs pour un comprimé de 350mg |
| Eudragit RS 30 D^{Ⓡ} (suspension aqueuse à 30% de matière sèche) | 45,67 mg |
| Eudragit RL 30 D^{Ⓡ} (suspension aqueuse à 30% de matière sèche) | 19,50 mg |
| Talc | 7,80 mg |
| Glycérol dibéhénate | 8,00 mg |
| Triéthylcitrate | 3,90 mg |
| Eau purifiée | qs |
| P.U.T | 350 mg exprimé en sec |

### Exemple 2 Un mode de mise en oeuvre du procédé selon la présente invention pour la préparation de comprimés dont la composition est donnée dans l'exemple 1.

### A) Mélange à sec

Dans un mélangeur FIELDER^{®}, introduire :
- sulfate ferreux
- maltodextrine
- cellulose microcristalline

Mélanger 10 min à basse vitesse afin d'obtenir un mélange homogène.

### B) Préparation de la suspension d'enrobage

Dans un récipient de capacité suffisante, introduire :
- 64% du mélange des Eudragit^{Ⓡ} dans un ratio massique Eudragit RS 30 D^{®} / Eudragit RL 30 D^{Ⓡ} égal à environ 65/35 ; préalablement filtrés sur tamis de 0,4 mm d'ouverture de maille.
- triéthylcitrate (20% de la quantité des Eudragit^{Ⓡ} exprimée en sec par comprimé).

Mélanger sous agitation à pâle modérée durant 10 minutes.

### C) Enrobage

Effectuer l'enrobage du mélange A) à l'aide de la suspension B).
- temps d'enrobage de la phase interne = 20 minutes environ.

Compléter l'enrobage si nécessaire avec de l'eau purifiée jusqu'à obtention d'une masse apte à être granulée.

### D) Emottage

Emotter la masse humide sur granulateur oscillant muni d'une grille de 3,15 mm d'ouverture de maille.

### E) Séchage

Sécher le granulé émotté sur lit d'air fluidisé jusqu'à obtention d'une humidité résiduelle du granulé séché comprise entre 2,5% et 3,5%.

### F) Calibrage

Calibrer le granulé séché en E) sur granulateur oscillant muni d'une grille de 1,5 mm d'ouverture de maille.

### G) Préparation de la suspension d'enrobage en lit d'air fluidisé

Dans un récipient de capacité suffisante, introduire :
- 36% du mélange des Eudragit^{®} dans un ratio massique Eudragit RS 30 D^{Ⓡ} / Eudragit RL 30 D^{Ⓡ} égal à environ 65/35 ; préalablement filtrés sur tamis de 0,4 mm d'ouverture de maille.

Mettre la suspension d'Eudragit^{Ⓡ} sous agitation puis introduire :
- talc
- triéthylcitrate (20% de la quantité des Eudragit^{Ⓡ} exprimée en sec par comprimé)

Laisser sous agitation jusqu'à parfaite homogénéité. H) Enrobage des granulés en lit d'air fluidisé

Introduire dans la cuve du lit d'air fluidisé, les granulés séchés et calibrés obtenu en F).

Pulvériser la suspension d'enrobage (à maintenir sous agitation durant toute la durée de l'enrobage) préparée en G) sur les granulés aux paramètres suivants :
- température air entrée : 40°C
- pression d'atomisation : 2 bars
- débit de pulvérisation : 30 grammes/min.
- diamètre buse pulvérisation : 1,2 mm.

Sécher les granulés enrobés précédemment en lit d'air fluidisé jusqu'à obtention d'une humidité résiduelle des granulés enrobés comprise entre 2,5% et 3,5%.

### I) Calibrage

Effectuer le calibrage des granulés enrobés sur granulateur oscillant muni d'une grille de 1,5 mm d'ouverture de maille.

### J) Lubrification

En fin de calibration, introduire dans le granulateur oscillant muni d'une grille de 1,5 mm d'ouverture de maille :
- talc
- dibéhénate de glycérol.

Mélanger 25 minutes.

L'échelle granulométrique ainsi obtenue est la suivante : 100% des granulés < 1,5 mm
30% des granulés > 0,71 mm
50% des granulés < 0,355 mm,
le diamètre moyen étant compris entre 0,25 mm et 0,355 mm.

### K) Compression

Comprimer les granulés lubrifiés sur presse rotative équipée de poinçons D9 R9.

### Paramètres pharmacotechniques :

- dureté moyenne (mesure effectuée sur Pharmatest^{Ⓡ} type PTB 301) : 63 N (maxi : 71 N, mini : 51 N)
- friabilité 100 tours (0,14%) (test de la friabilité des comprimés selon la monographie 2.9.7 de la Pharmacopée Européenne, édition en vigueur) .

## Revendications

1. Composition pharmaceutique ou nutraceutique comprenant au moins un granulé enrobé, à libération prolongée de sel de fer; ledit granulé enrobé étant composé d'une particule comprenant ledit sel de fer enrobée d'au moins deux enveloppes **caractérisées en ce qu'**elles comprennent une combinaison d'excipients composée :
- d'au moins un copolymère (a) : poly(ethyl acrylate-co-methyl methacrylate-co-trimethylammonioethyl methacrylate chloride) 1:2:0.1 (Eudragit RS30D^{Ⓡ}) présentant un pourcentage molaire de groupes ammonium quaternaire inférieur ou égal à 8%,
- en association avec un second copolymère (b) : poly(ethyl acrylate-co-methyl methacrylate-co-trimethylammonioethyl methacrylate chloride) 1:2:0.2 (Eudragit RL30D^{Ⓡ}) présentant un pourcentage molaire de groupes ammonium quaternaire supérieur à 8%,
- dans un ratio massique (a) / (b) compris entre 60/40 et 80/20,
- et avec une quantité de (a) comprise entre 2,5% et 5,0% en poids sec du poids total de la composition.

2. Composition selon la revendication 1 **caractérisée en ce que** le sel de fer est sous forme de fer II ou III.

3. Composition selon la revendication 2 **caractérisée en ce que** le sel de fer est choisi parmi le groupe constitué du sulfate, fumarate, gluconate, ascorbate, oxalate, succinate, glycérophosphate et férédétate.

4. Composition selon la revendication 1 **caractérisée en ce que** le sel de fer est du sulfate ferreux.

5. Composition selon la revendication 1 **caractérisée en ce qu'**elle ne comprend pas d'antioxydant.

6. Composition selon la revendication 1 **caractérisée en ce que** le pourcentage molaire de groupements ammonium quaternaire dans le copolymère (a) est compris entre 2% et 8% et préférentiellement 5%.

7. Composition selon la revendication 1 **caractérisée en ce que** le pourcentage molaire de groupements ammonium quaternaire dans le copolymère (b) est compris entre 8 et 12% et préférentiellement 10%.

8. Composition selon la revendication 1 **caractérisée en ce que** le ratio massique (a) / (b) dans la composition finale est 70/30, et préférentiellement 65/35.

9. Composition selon la revendication 1 **caractérisée en ce que** la quantité de copolymère (a) est comprise entre 3,5% et 4,0% en poids sec du poids total de la composition.

10. Composition selon la revendication 1 **caractérisée en ce que** la combinaison d'excipients comprend un diluant, un liant, un lubrifiant, un anti-agglomérant, un plastifiant utilisés seuls ou en mélange.

11. Composition selon la revendication 10 **caractérisée en ce que** le plastifiant est choisi dans le groupe formé de l'acétyl tributyl citrate, acétyl triéthyl citrate, glycérides d'acides gras acétylés, huile de ricin, diéthyl sébacate, dibutyl sébacate, glycérol, monostéarate de glycérol, glycéryl triacétate, polyéthylène glycols, copolymères polyoxyéthylène/polyoxypropylène, propylène glycol, tributyl citrate, triéthylcitrate utilisés seuls ou en mélange et préférentiellement du triéthylcitrate.

12. Composition selon la revendication 10 **caractérisée en ce que** le diluant est choisi dans le groupe formé des celluloses et de lactose et préférentiellement une cellulose.

13. Composition selon la revendication 10 **caractérisée en ce que** le liant est choisi dans le groupe formé des maltodextrines et des povidones et préférentiellement une maltodextrine.

14. Utilisation d'une composition selon les revendications 1 à 13 pour la préparation d'un produit pharmaceutique ou nutraceutique, à libération prolongée de sel$ de fer.

15. Utilisation d'une composition selon les revendications 1 à 13 pour la préparation d'un produit pharmaceutique, nutraceutique, cosmétique ou vétérinaire destiné au traitement et/ou à la prévention des carences en minéraux et oligoéléments.

16. Utilisation selon la revendication 14 **caractérisée en ce que** la préparation pharmaceutique, nutraceutique, cosmétique ou vétérinaire est sous forme orale.

17. Utilisation selon la revendication 16 **caractérisée en ce que** la forme orale est choisie parmi des comprimés, des gélules, des capsules, des pastilles à sucer, des poudres pour suspensions buvables et pour sirops.

18. Utilisation d'une composition selon les revendications 3 à 13 pour la préparation d'un médicament destiné au traitement et/ou à la prévention des carences en fer avec ou sans anémie.

19. Utilisation d'une composition selon les revendications 3 à 13 pour la préparation d'un médicament destiné au traitement et/ou à la prévention de l'anémie par carence martiale.

20. Utilisation d'une composition selon les revendications 3 à 13 pour la préparation d'un médicament destiné au traitement et/ou à la prévention de la carence martiale de la femme enceinte, lorsqu'un apport alimentaire suffisant en fer ne peut être assuré.

21. procédé de préparation d'une composition pharmaceutique ou nutraceutique selon les revendications 1 à 13, **caractérisé en ce qu'**il comprend les étapes suivantes :
1) Enrobage du sel de fer avec une dispersion aqueuse des deux copolymères (a) et (b), dont le ratio massique (a) / (b) est compris entre 60/40 et 80/20, et avec une quantité de (a) comprise entre 2,5% et 5,0% en poids sec du poids total de la composition ; dans un mélangeur à pâles,
2) Emottage du granulé obtenu en 1),
3) Séchage des granulés obtenus en 2),
4) Enrobage en lit d'air fluidisé des granulés séchés obtenus en 3) par pulvérisation d'une dispersion aqueuse des deux copolymères (a) et (b), dont le ratio massique (a) / (b) est compris entre 60/40 et 80/20, et avec une quantité de (a) comprise entre 3,5% et 5,0% en poids sec du poids total de la composition,
5) Séchage des granulés enrobés obtenus en 4).

22. Procédé selon la revendication 21 **caractérisé en ce qu'**il ne fait pas intervenir de solvant organique.

23. Procédé selon la revendication 21 **caractérisé par** un ratio massique (a) / (b) identique lors des étapes 1) et 4).

24. Procédé selon la revendication 21 **caractérisé en ce que** les granulés obtenus sont directement conditionnés sous forme de poudre pour suspension buvable ou pour sirop.

25. Procédé selon la revendication 21 **caractérisé en ce qu'**il comprend une étape supplémentaire de lubrification des granulés pour l'obtention de comprimés, de gélules et de pastilles à sucer.

26. Procédé selon la revendication 21 **caractérisé en ce qu'**il comprend une étape supplémentaire de compression des granulés pour l'obtention de comprimés et de pastilles à sucer.

27. Procédé selon la revendication 21 **caractérisé en ce que** l'étape 4) est réalisée plusieurs fois successivement.

## Claims

1. Pharmaceutical or nutraceutical composition comprising at least one coated granule, with sustained release of an iron salt; said coated granule being composed of a particle that comprises said iron salt and is coated with at least two coatings, **characterised in that** they comprise a combination of excipients composed:
- of at least one copolymer (a): poly(ethyl acrylate-co-methyl methacrylate-co-trimethylammonioethyl methacrylate chloride) 1:2:0.1 (Eudragit RS30D^{®}) having a molar percentage of quaternary ammonium groups of less than or equal to 8%,
- in combination with a second copolymer (b): poly(ethyl acrylate-co-methyl methacrylate-co-trimethylammonioethyl methacrylate chloride) 1:2:0.2 (Eudragit RL30D^{®}) having a molar percentage of quaternary ammonium groups of greater than 8%,
- in a ratio by weight (a)/(b) of from 60/40 to 80/20,
- and with an amount of (a) of from 2.5% to 5.0% by dry weight, based on the total weight of the composition.

2. Composition according to Claim 1, **characterised in that** the iron salt is in the form of iron(II) or iron(III).

3. Composition according to Claim 2, **characterised in that** the iron salt is selected from the group comprising the sulfate, fumarate, gluconate, ascorbate, oxalate, succinate, glycerophosphate and feredetate.

4. Composition according to Claim 1, **characterised in that** the iron salt is iron(II) sulfate.

5. Composition according to Claim 1, **characterised in that** it does not comprise any antioxidant.

6. Composition according to Claim 1, **characterised in that** the molar percentage of quaternary ammonium groups in copolymer (a) is from 2% to 8% and is preferably 5%.

7. Composition according to Claim 1, **characterised in that** the molar percentage of quaternary ammonium groups in copolymer (b) is from 8% to 12% and is preferably 10%.

8. Composition according to Claim 1, **characterised in that** the ratio by weight (a)/(b) in the final composition is 70/30 and preferably 65/35.

9. Composition according to Claim 1, **characterised in that** the amount of copolymer (a) is from 3.5% to 4.0% by dry weight, based on the total weight of the composition.

10. Composition according to Claim 1, **characterised in that** the combination of excipients comprises a diluent, a binder, a lubricant, an anti-agglomeration agent, a plasticizer, used on their own or in the form of a mixture.

11. Composition according to Claim 10, **characterised in that** the plasticizer is selected from the group formed by acetyl tributyl citrate, acetyl triethyl citrate, acetylated fatty acid glycerides, castor oil, diethyl sebacate, dibutyl sebacate, glycerol, glycerol monostearate, glyceryl triacetate, polyethylene glycols, polyoxyethylene/polyoxypropylene copolymers, propylene glycol, tributyl citrate, triethyl citrate, used on their own or in the form of a mixture, and is preferably triethyl citrate.

12. Composition according to Claim 10, **characterised in that** the diluent is selected from the group formed by celluloses and lactose and is preferably a cellulose.

13. Composition according to Claim 10, **characterised in that** the binder is selected from the group formed by maltodextrins and povidones and is preferably a maltodextrin.

14. Use of a composition according to Claims 1 to 13 in the preparation of a pharmaceutical or nutraceutical product with sustained release of iron salt.

15. Use of a composition according to Claims 1 to 13 in the preparation of a pharmaceutical, nutraceutical, cosmetic or veterinary product for the treatment and/or prevention of deficiencies of minerals and oligoelements.

16. Use according to Claim 14, **characterised in that** the pharmaceutical, nutraceutical, cosmetic or veterinary preparation is in oral form.

17. Use according to Claim 16, **characterised in that** the oral form is selected from tablets, gelatin capsules, capsules, pastilles for sucking, and powders for drinkable suspensions and for syrups.

18. Use of a composition according to Claims 3 to 13 in the preparation of a medicament for the treatment and/or prevention of iron deficiencies with or without anaemia.

19. Use of a composition according to Claims 3 to 13 in the preparation of a medicament for the treatment and/or prevention of iron-deficiency anaemia.

20. Use of a composition according to Claims 3 to 13 in the preparation of a medicament for the treatment and/or prevention of iron deficiency in pregnant women, when a sufficient dietary intake of iron cannot be ensured.

21. Process for the preparation of a pharmaceutical or nutraceutical composition according to Claims 1 to 13, **characterised in that** it comprises the following steps:
1) the iron salt is coated, in a blade mixer, with an aqueous dispersion of the two copolymers (a) and (b), the ratio by weight (a)/(b) of which is from 60/40 to 80/20, with an amount of (a) of from 2.5% to 5.0% by dry weight, based on the total weight of the composition,
2) the granule obtained in 1) is broken up,
3) the granules obtained in 2) are dried,
4) the dried granules obtained in 3) are coated in a fluidized-air bed by atomization of an aqueous dispersion of the two copolymers (a) and (b), the ratio by weight (a)/(b) of which is from 60/40 to 80/20, with an amount of (a) of from 3.5% to 5.0% by dry weight, based on the total weight of the composition,
5) the coated granules obtained in 4) are dried.

22. Process according to Claim 21, **characterised in that** it does not involve the use of an organic solvent.

23. Process according to Claim 21, **characterised in that** the ratio by weight (a)/(b) in steps 1) and 4) is identical.

24. Process according to Claim 21, **characterised in that** the granules obtained are packaged directly in the form of powder for a drinkable suspension or for syrup.

25. Process according to Claim 21, **characterised in that** it comprises, to give tablets, gelatin capsules or pastilles for sucking, an additional step in which the granules are lubricated.

26. Process according to Claim 21, **characterised in that** it comprises an additional step in which the granules are compressed to give tablets or pastilles for sucking.

27. Process according to Claim 21, **characterised in that** step 4) is carried out several times in succession.

## Patentansprüche

1. Pharmazeutische oder nutrazeutische Zusammensetzung, die mindestens ein beschichtetes Korn umfasst, zur verlängerten Freisetzung von Eisensalz; wobei das beschichtete Korn aus einem das Eisensalz umfassenden Teilchen besteht, das mit mindestens zwei Überzügen beschichtet ist, **dadurch gekennzeichnet, dass** die Überzüge eine Kombination von Hilfsstoffen aus:
- mindestens einem Copolymer (a): Poly(ethylacrylat-co-methylmethacrylat-co-trimethylammonioethylmethacrylatchlorid) 1:2:0,1 (Eudragit RS30D^{®}) mit einem Molprozentanteil an quartären Ammoniumgruppen kleiner gleich 8%
- in Kombination mit einem zweiten Copolymer (b): Poly(ethylacrylat-co-methylmethacrylat-co-trimethylammonioethylmethacrylatchlorid) 1:2:0,2 (Eudragit RL30D^{®}) mit einem Molprozentanteil an quartären Ammoniumgruppen von mehr als 8%
- in einem Gewichtsverhältnis (a) / (b) zwischen 60/40 und 80/20
- und mit einer Menge von (a) zwischen 2,5 und 5,0 Trockengew.-%, bezogen auf das Gesamtgewicht der Zusammensetzung,
umfassen.

2. Zusammensetzung nach Anspruch 1, **dadurch gekennzeichnet, dass** das Eisensalz in Form von Eisen(II) oder Eisen(III) vorliegt.

3. Zusammensetzung nach Anspruch 2, **dadurch gekennzeichnet, dass** das Eisensalz aus der Gruppe bestehend aus Sulfat, Fumarat, Gluconat, Ascorbat, Oxalat, Succinat, Glycerophosphat und Feredetat ausgewählt ist.

4. Zusammensetzung nach Anspruch 1, **dadurch gekennzeichnet, dass** es sich bei dem Eisensalz um Eisen(II)-sulfat handelt.

5. Zusammensetzung nach Anspruch 1, **dadurch gekennzeichnet, dass** sie kein Antioxidans umfasst.

6. Zusammensetzung nach Anspruch 1, **dadurch gekennzeichnet, dass** der Molprozentanteil an quartären Ammoniumgruppen in dem Copolymer (a) zwischen 2 und 8% und vorzugsweise 5% liegt.

7. Zusammensetzung nach Anspruch 1, **dadurch gekennzeichnet, dass** der Molprozentanteil an quartären Ammoniumgruppen in dem Copolymer (b) zwischen 8 und 12% und vorzugsweise 10% liegt.

8. Zusammensetzung nach Anspruch 1, **dadurch gekennzeichnet, dass** das Gewichtsverhältnis (a) / (b) in der Endzusammensetzung 70/30 und vorzugsweise 65/35 beträgt.

9. Zusammensetzung nach Anspruch 1, **dadurch gekennzeichnet, dass** die Menge von Copolymer (a) zwischen 3,5 und 4,0 Trockengew.-%, bezogen auf das Gesamtgewicht der Zusammensetzung, liegt.

10. Zusammensetzung nach Anspruch 1, **dadurch gekennzeichnet, dass** die Kombination von Hilfsstoffen ein Verdünnungsmittel, ein Bindemittel, ein Gleitmittel, ein Antiagglomerationsmittel und/oder einen Weichmacher umfasst.

11. Zusammensetzung nach Anspruch 10, **dadurch gekennzeichnet, dass** der Weichmacher aus der Gruppe bestehend aus Acetyltributylcitrat, Acetyltriethylcitrat, acetylierten Fettsäureglyceriden, Ricinusöl, Diethylsebacat, Dibutylsebacat, Glycerin, Glycerinmonostearat, Glycerintriacetat, Polyethylenglykolen, Polyoxyethylen/polyoxypropylen-Copolymeren, Propylenglykol, Tributylcitrat und/oder Triethylcitrat ausgewählt ist und vorzugsweise Triethylcitrat ist.

12. Zusammensetzung nach Anspruch 10, **dadurch gekennzeichnet, dass** das Verdünnungsmittel aus der Gruppe bestehend aus Cellulosen und Lactose ausgewählt ist und vorzugsweise eine Cellulose ist.

13. Zusammensetzung nach Anspruch 10, **dadurch gekennzeichnet, dass** das Bindemittel aus der Gruppe bestehend aus Maltodextrinen und Povidonen ausgewählt ist und vorzugsweise ein Maltodextrin ist.

14. Verwendung einer Zusammensetzung nach den Ansprüchen 1 bis 13 zur Herstellung eines pharmazeutischen oder nutrazeutischen Produkts zur verlängerten Freisetzung von Eisensalz.

15. Verwendung einer Zusammensetzung nach den Ansprüchen 1 bis 13 zur Herstellung eines pharmazeutischen, nutrazeutischen, kosmetischen oder veterinärmedizinischen Produkts zur Behandlung und/oder Prävention von Mineralien- und Spurenelementemängeln.

16. Verwendung nach Anspruch 14, **dadurch gekennzeichnet, dass** das pharmazeutische, nutrazeutische, kosmetische oder veterinärmedizinische Präparat in oraler Form vorliegt.

17. Verwendung nach Anspruch 16, **dadurch gekennzeichnet, dass** die orale Form unter Tabletten, Kapseln einschließlich Gelatinekapseln, Lutschpastillen und Pulvern für trinkbare Suspensionen und für Sirupe ausgewählt ist.

18. Verwendung einer Zusammensetzung nach den Ansprüchen 3 bis 13 zur Herstellung eines Arzneimittels zur Behandlung und/oder Prävention von Eisenmängeln mit oder ohne Anämie.

19. Verwendung einer Zusammensetzung nach den Ansprüchen 3 bis 13 zur Herstellung eines Arzneimittels zur Behandlung und/oder Prävention von Eisenmangelanämie.

20. Verwendung einer Zusammensetzung nach den Ansprüchen 3 bis 13 zur Herstellung eines Arzneimittels zur Behandlung und/oder Prävention von Eisenmangel bei Schwangeren, wenn keine ausreichende Eisenzufuhr über die Nahrung gewährleistet werden kann.

21. Verfahren zur Herstellung einer pharmazeutischen oder nutrazeutischen Zusammensetzung nach den Ansprüchen 1 bis 13, dadurch gekenntzeichnet, dass es die folgenden Schritte umfasst:
1) Beschichten des Eisensalzes mit einer wässrigen Dispersion der beiden Copolymere (a) und (b), wobei das Gewichtsverhältnis (a) / (b) zwischen 60/40 und 80/20 liegt und die Menge von (a) zwischen 2,5 und 5,0 Trockengew.-%, bezogen auf das Gesamtgewicht der Zusammensetzung, liegt; in einem Schaufelmischer,
2) Aufbrechen des in 1) erhaltenen Korns,
3) Trocknen der in 2) erhaltenen Körner,
4) Beschichten der in 3) erhaltenen getrockneten Körner durch Zerstäuben einer wässrigen Dispersion der beiden Copolymere (a) und (b), wobei das Gewichtsverhältnis (a) / (b) zwischen 60/40 und 80/20 liegt und die Menge von (a) zwischen 3,5 und 5,0 Trockengew.-%, bezogen auf das Gesamtgewicht der Zusammensetzung, liegt, im Luftwirbelbett,
5) Trocknen der in 4) erhaltenen beschichteten Körner.

22. Verfahren nach Anspruch 21, **dadurch gekennzeichnet, dass** dabei kein organisches Lösungsmittel verwendet wird.

23. Verfahren nach Anspruch 21, **dadurch gekennzeichnet, dass** das Gewichtsverhältnis (a) / (b) bei den Schritten 1) und 4) identisch ist.

24. Verfahren nach Anspruch 21, **dadurch gekennzeichnet, dass** die erhaltenen Körner direkt in Form von Pulver für eine trinkbare Suspension oder einen Sirup konditioniert werden.

25. Verfahren nach Anspruch 21, **dadurch gekennzeichnet, dass** es einen zusätzlichen Schritt der Lubrifizierung der Körner zum Erhalt von Tabletten, Gelatinekapseln und Lutschpastillen umfasst.

26. Verfahren nach Anspruch 21, **dadurch gekennzeichnet, dass** es einen zusätzlichen Schritt des Verpressens der Körner zum Erhalt von Tabletten und Lutschpastillen umfasst.

27. Verfahren nach Anspruch 21, **dadurch gekennzeichnet, dass** Schritt 4) mehrmals aufeinanderfolgend durchgeführt wird.
